# EUROPÄISCHE PATENTSCHRIFT

(11) **EP 0 347 493 B1**
(45) Veröffentlichungstag und Bekanntmachung des Hinweises auf die Patenterteilung: **10.03.1993**
(21) Anmeldenummer: 88118469.1
(22) Anmeldetag: 05.11.1988
(51) Int. Cl.: A61K 35/78, A23L 1/221

(54) **Verfahren zur Herstellung eines die wasserdampfflüchtigen und andere lipophile Inhaltsstoffe enthaltenden Teilextraktes aus Heil- und/oder Gewürzpflanzen**
Process for producing partial medicinal and/or herbal plant extracts containing vapour-volatile and other lipophilic constituents
Procédé de préparation d'un extrait partiel de plantes médicinales et/ou d'épices, contenant des constituants entraînés à la vapeur d'eau et des constituants lipophiles

(30) Priorität: 24.06.1988 CH 2441/88
(43) Veröffentlichungstag der Anmeldung: 27.12.1989
(73) Patentinhaber: Emil Flachsmann AG, CH-8038 Zürich (CH)
(72) Erfinder: Honerlagen, Hans J., CH-6314 Unterägeri (CH); Steiner, Rudolf, CH-8303 Bassersdorf (CH)
(74) Vertreter: Zink, Markus, Dr.

(56) Entgegenhaltungen:
- EP-A- 0 006 060
- EP-A- 0 062 893
- EP-A- 0 065 222
- CH-A- 631 624
- DE-A- 626 469
- DE-A- 943 250
- DE-A- 2 627 534
- US-A- 2 571 948
- US-A- 4 198 432

## Beschreibung

Die vorliegende Erfindung betrifft ein Verfahren zur Herstellung eines die wasserdampfflüchtigen und andere lipophile Inhaltsstoffe enthaltenden Teilextraktes aus Heil- und/oder Gewürzpflanzen oder Teilen davon, welche im frischen oder getrockneten Zustand eingesetzt werden können.

Die Erfindung betrifft auch ein Verfahren zur Herstellung eines die wasserdampfflüchtigen und andere lipophile sowie die hydrophilen Inhaltsstoffe enthaltenden Vollextraktes aus Heil- und/oder Gewürzpflanzen oder Teilen davon, welche im frischen oder getrockneten Zustand eingesetzt werden können.

Ueblicherweise werden Pflanzenextrakte durch Extraktion mit Alkoholen, Mischungen aus Alkoholen und Wasser oder auch nur mit Wasser hergestellt. In ganz wenigen Ausnahmen werden chlorierte Kohlenwasserstoffe oder niedrig siedende Kohlenwasserstoffe, wie z.B. bei der Hopfenextraktion für die Brauwirtschaft, eingesetzt.

In allen Fällen werden aber nur bestimmte Inhaltsstoffgruppen extrahiert, so dass man von Vollextrakten, welche das gesamte Inhaltsstoffspektrum der eingesetzten Pflanze enthalten, nicht sprechen kann.

Es ist auch bekannt, dass frische Pflanzen schon beim Zerkleinern enzymatische Aktivitäten entwickeln und somit schon, bevor sie überhaupt extrahiert werden können, entweder ganz oder teilweise ihre nativen Inhaltsstoffe verlieren. Als Beispiel sei hier der frische Knoblauch (Bulbus Alii sativi) angeführt. Bisher ist es nicht gelungen, einen Knoblauchextrakt herzustellen, welcher das Alliin in unveränderter Form enthält. Siehe hierzu Miething H., Thober H., Apotheker Journal 10, Seiten 42-48 (1985) und Koch H.P., Deutsche Apotheker Zeitung 127, 8, Seiten 367-369 (1987). Das Allicin, welches seit ca. 40 Jahren als wirksames Prinzip des Knoblauchs gilt, entsteht sofort aus der genuinen Vorstufe, dem Alliin, beim Zerkleinern der Droge druch Einwirkung des Enzyms Alliinase; siehe hierzu Koch H.P., Deutsche Apotheker Zeitung 127, 8, Seiten 367-369 (1987). In den intakten Knoblauchzehen liegen diese beiden Substanzen in getrennten Zellen vor; bei der Zerstörung der Zellwände reagieren beide Substanzen blitzartig miteinander. Siehe Block E., Spektrum der Wissenschaft (Mai 1985) Seiten 66-72.

Der vorliegende Erfindung lag nun die Aufgabe zugrunde, Konzentrate von Teil- und Vollextrakten von Heil- und/oder Gewürzpflanzen zur Verfügung zu stellen, welche auch die wasserdampfflüchtigen Inhaltsstoffe enthalten. Es sollen auch Extrakte zur Verfügung gestellt werden, welche stabil sind und die nativen Stoffe enthalten. Diese Aufgaben sollen mittels einem einfachen, billigen und wirtschaftlichen Verfahren realisiert werden.

Es wurde nun völlig überraschend gefunden, dass durch Extraktion mit wenigstens einem organischen Lösungsmittel, z. B. mit niederen Alkoholen und Ketonen, die wasserdampfflüchtigen Bestandteile von Heil- und Gewürzpflanzen oder Teilen davon im nativen Zustand extrahiert und erhalten werden können, wenn man während der Extraktion dem Lösungsmittel das durch die Pflanze eingebrachte Wasser entzieht oder das Extrakt, auch Menstruum genannt, vor der Eindampfung entwässert.

Es wurde weiterhin völlig überraschend gefunden, dass Pflanzen oder Teile davon, die schon beim Zerkleinern enzymatische Aktivitäten entwickeln und somit schon, bevor sie überhaupt extrahiert werden können, entweder ganz oder teilweise ihre nativen Inhaltsstoffe verloren haben, durch Zerkleinerung unter wenistens einem organischen Lösungsmittel, z.B. unter niederen Alkoholen oder Ketonen, ihre Enzymaktivitäten nicht entfalten können und somit die Produktion von Extrakten erlauben, welche stabil sind und die nativen Stoffe enthalten. Voraussetzung hierfür ist, dass entweder die Lösungsmittelmenge so gewählt wird, dass das von der Pflanze, auch Droge genannt, eingebrachte Wasser so gering ist, dass keine Enzymaktivitäten möglich sind, oder aber dem Extraktionsmittel sofort das Wasser entzogen wird.

Die Erfindung ist durch die Merkmale in den unabhängigen Ansprüchen gekennzeichnet. Bevorzugte Ausführungsformen sind in den abhängigen Ansprüchen definiert.

Im erfindungsgemässen Verfahren kann in der organischen Phase das Wasser auch mittels Membrantechnologie anstelle der Verwendung eines Trocknungsmittels abgetrennt werden.

Werden nun Knoblauchzehen unter einem organischen Lösungsmittel zerkleinert, so wird die Reaktion zwischen dem Alliin und dem Enzym Alliinase unterbunden.

Mit den erfindungsgemässen Verfahren werden insbesondere folgende Plfanzen extrahiert:
Acorus calamus L. (Rhizom)
Allium - Arten (z.B.:A. cepa L., A. ursinum L., A. sativum L.: Bulbus)
Alpinia officinarum Hance (Rhizom)
Anethum graveolens L. (Fructus)
Angelica archangelica L. div. Subspec. (Rhizoma)
Anthemis nobilis L. (Chamomilla romana, Herba)
Apium graveolens L. (Fructus)
Arctium major Gaertn. (Radix)
Arnica montana L. (Flos)
Artemisia absinthium L. (Herba)
Artemisia dracunculus L. (Herba)
Brassica nigra [L.] Koch (Semen)
Carum carvi L. (Fructus)
Chrysanthemum vulgare Asch. (Herba)
Cinnamomum camphora, div. Species (Cortex)
Cinnamomum ceylanicum Nees. (Cortex)
Citrus: div. Species (Folium, Flavedo, Fruct.)
Copaifera reticulata Duke (Balsam)
Coriandrum sativum L. (Fructus)
Cuminum cyminum L. (Fructus)
Curcuma zedoaria [Bergius] Roxb. u. andere Unterarten (Rhizoma)
Cusparia officinalis [Willd.] Eng. (Cortex)
Dipterocarpus turbinatus Gaertn. (Balsamum)
Drosera - Arten (D. rotundifolia L., D.ramentacea Burch; Herba)
Elettaria cardamomum [L.] White et Mathon (Fructus)
Eucalyptus globulus Labill. (Folium)
Foeniculum vulgare Miller (Fructus)
Gaultheria procumbens L. (Folium)
Hedoma pulegioides [L.] Pers. (Herba)
Hibiscus abelmoschus L. (Semen)
Humulus lupulus L. (Flos, Glandulae)
Hysopus officinalis L. (Herba)
Illicium verum Hook. f. (Fructus)
Inula helenium L. (Rhizoma)
Iris Pallida Lam. (Rhizoma)
Jasminum grandiflorum L. (Flos)
Laurus nobilis L. (Folium, Fructus)
Lavendula officinalis, div. subspecies (Flos)
Lawsonia inermis L. (Folium)
Levisticum officinale Koch (Radix)
Matricaria chamomilla L. (Flos)
Melaleuca: div. Variet. (Folium)
Melilotus officinalis [L.] Lam. em. Thuill. (Herba)
Melissa officinalis L. (Herba)
Mentha, alle Varietäten (Folium)
Myristica fragrans Houttuyn (Arillus, Semen)
Myrtus communis L. (Folium)
Ocimum basilicum L. (Herba)
Ocotea sassafras (Cortex)
Oenanthe aquatica [L.] Poir (Fructus)
Olibanum (Resinum)
Origanum majorana L. u. andere Unterarten (Herba)
Petroselinum crispum [Mill.] Nym. (Fructus, Herba)
Pimenta dioica [L.] Merill (Fructus)
Pimpinella anisum L. (Semen)
Piper angustifolium Ruiz. et Pavon. (Folium)
Pogostemon patchouli Pell. (Folium)
Prunus laurocerasus L. (Folium)
Rosmarinus officinalis L. u a. Unterarten (Folium)
Ruta graveolens L. (Herba)
Salvia officinalis L. u.a. Unterarten (Folium)
Santalum album L. (Lignum)
Sarothamnus scoparius [L.] Wimmer (Herba)
Sassafras albidum [Nutt.] Nees (Lignum)
Satureja hortensis L. (Herba)
Syzygium aromaticum Merr. et Perry (Flores, Folium)
Thymus serpyllum L. (Herba)
Thymus vulgaris L. (Herba)
Tilia cordata Mill. und T platyphyllos Scop. (Flos)
Valeriana officinalis u.andere Unterarten (Radix)
Zingiberis officinale Roscoe (Rhizoma).

Pflanzen mit ätherischen Oelen wie z.B.
- Fructus anisi stellati
- Fructus anisi vulg.
- Fructus carvi
- Flores humuli lup.
- Fructus coriandri
- Fructus juniperi
- Fructus foeniculi
- Flores chamomillae
- Herba thymi
- Radix valerianae offic.
- Rhiz. zingiberis

werden mit niederen Alkoholen oder Ketonen nach bekannten Extraktionsverfahren erschöpfend extrahiert, das Menstruum vor der Eindampfung entwässert. Die Konzentrierung des entwässerten Menstruums erfolgt zweckmässigerweise unter Vakuum bis zur Lösungsmittelfreiheit. Erhalten werden die lipophilen Bestandteile der Pflanze einschliesslich der ätherischen Oele. So wird z. B. bei der Kamille das Matricin erhalten, die Vorstufe des nicht sehr stabilen Azulens.

Die so extrahierten Pflanzen werden dann mit kaltem oder heissem Wasser oder mit Wasser mischbaren, verdünnten organischen Lösungsmitteln extrahiert.

Dieser Extrakt enthält dann alle polaren Inhaltsstoffe der Pflanze. Das Menstruum wird ebenfalls unter vermindertem Druck bis zur Sirupkonsistenz eingedampft und nach bekannten Verfahren getrocknet.

Durch Vereinigung der beiden Phasen, empfehlenswerterweise in der Endformulierung, werden Vollextrakte erhalten, die das Wirkungsspektrum der Heil- oder Gewürzpflanze abdecken.

Pflanzen ohne ätherische Oele aber mit wasserdampfflüchtigen Substanzen wie z.B.
- Herba droserae
- Folia henna
- Herba spartii scopar.
- Herba meliloti offic.

werden ebenfalls mit niederen Alkoholen oder niederen Ketonen extrahiert, das Menstruum vor der Eindampfung entwässert, so dass die wasserdampfflüchtigen Verbindungen vollständig im Eindampfkonzentrat erhalten bleiben. Die so von ihren wasserdampfflüchtigten Verbindungen befreiten Drogen werden mit Wasser extrahiert.

Nach getrennter Trocknung der beiden Phasen werden beide zu einem Vollextrakt vereinigt.

Als geeignetes Mittel zur Entwässerung der organischen Phasen hat sich wasserfreies Natriumsulfat, Gelatine oder Traganth bewährt.

Werden die Rückstände mit Wasser extrahiert, so versteht es sich von selbst, dass das vorgängig eingebrachte Trocknungsmittel nicht wasserlöslich sein darf. Man verwendet dann z. B. Traganth oder Molekularsiebe.

Die erfindungsgemässen hergestellten Teil- oder Vollextrakte, vorzugsweise in Form eines lipophilen Konzentrates, oder eines die lipophilen und hydrophilen Inhaltsstoffe enthaltenen Konzentrates, können in Abhängigkeit von der jeweils extrahierten Pflanze als Heilmittel oder als Gewürz verwendet werden.

Die nachfolgenden Beispiele sollen die Erfindung illustrieren.

### Beispiel 1

10 kg gemahlene Baldrianwurzeln (Radix valerianae offic. 1.) wurden mit 5 Liter 96%igem Ethanol durchfeuchtet und über Nacht stehen gelassen. Am nächsten Morgen wurde die feuchte Droge in ein Perkolationsrohr gefüllt und dann mit 75 Liter 96%igem Ethanol bei Raumtemperatur perkoliert. Das Perkolat wurde durch Zusatz von 3 kg Gelatinepulver entwässert und durch Filtration von der wasserhaltigen Gelatine befreit. Das Filtrat wurde bei max. 40°C und vermindertem Druck vom Lösungsmittel befreit. Erhalten wurden 0,34 kg mit einem ätherischen Oelgehalt von 5,3 Gew.-%. Die mit Ethanol extrahierte Droge wurde an der Luft getrocknet und dann mit 80 Liter heissem Wasser von ca. 90°C im Rührverfahren während 60 Minuten extrahiert. Dann wurde filtriert und das Filtrat unter reduziertem Druck bei max. 70°C zur Sirupkonsistenz eingeengt. Der sirupöse Extrakt wurde dann im Vakuum-Trockenschrank bei max. 70°C getrocknet. Erhalten wurden 1,25 kg Trockenextrakt.

Als Darreichungsform wurden Hartgelatine-Kapseln gewählt. In jede Kapsel wurden 68 mg der mit Ethanol gewonnenen Phase und 249,5 mg der Wasserphase gegeben, so dass in jeder Kapsel die Extraktivstoffe aus 2,0 g Baldrianwurzeln enthalten sind.

Die Deutsche Monographie der Kommission E schreibt für Baldrianwurzeln eine Einzeldosis an Extrakt von 2-3 g Droge entsprechend vor.

Durch Einnahme einer Kapsel wird die Einzeldosis erreicht.

### Beispiel 2

10 kg Hopfenblüten (Flores humuli lupuli) wurden wie unter Beispiel 1 beschreieben extrahiert.

Erhalten wurden 1,6 kg lösungsmittelfreie Ethanolphase mit einem ätherischen Oelgehalt von 2,3 Gew.-%.

An getrockneter Wasserphase wurden 1,5 kg erhalten.

Auch hier wurden Hartgelatine-Kapseln hergestellt.

In jeder Kapsel wurden 160 mg der mit Ethanol gewonnenen Phase und 150 mg der mit Wasser gewonnen Phase gegeben.

Die Deutsche Standardzulassung für Hopfenblüten schreibt eine Einzeldosis von 1-2 Teelöffel Hopfenzapfen zur Bereitung einer Tasse Tee vor. 1 Teelöffel Hopfenblüten wiegt 1 g, so dass die beschriebene Kapsel einer Einzeldosis entspricht.

### Beispiel 3

30 kg frische Knoblauchzehen wurden in 400 kg Methanol gegeben.

Die Zerkleinerung der Knoblauchzehen erfolgte unter Methanol langsam und vorsichtig mittels einer Schneidvorrichtung. Nach Zerkleinerung sämtlicher Knoblauchzehen wurde noch eine Stunde bei Raumtemperatur unter Rühren extrahiert.

Dann wurde filtriert, dem Filtrat wurde 16 kg wasserfreies Natriumsulfat zugesetzt und 2 Stunden gerührt. Nach 2 Stunden wurde filtriert und das wasserfreie Filtrat unter vermindertem Druck bei max. 40°C zur Sirupkonsistenz eingeengt. Der sirupöse Extrakt wurde dann bei max. 70°C im Vakuum-Trockenschrank getrocknet. Erhalten wurde 1 kg Trockenextrakt mit ca. 8 Gew.-% Alliin. Die mit Methanol extrahierte Droge wurde an der Luft getrocknet und mit 240 Liter Wasser 1 Stunde bei 90°C extrahiert. Nach Abtrennung des Drogenrückstandes wurde das wässrige Menstruum bei max. 70°C unter vermindertem Druck zur Sirupkonsistenz eingedampft und dann im Vakuum-Trockenschrank getrocknet. Erhalten wurden 5 kg Trockenextrakt.

Durch Vermischung von 1 Teil der mit Methanol gewonnen Phase und 5 Teilen der mit Wasser gewonnenen Phase wird ein Vollextrakt aus Knoblauchzehen erhalten.

Durch diese Verfahrensweise wird eine Konzentrierung auf das fünffache der frischen Knoblauchzehen erreicht.

Wenn aus therapeutischer Sicht aber nur Wert auf die alliinhaltige Phase gelegt wird, so ist hier eine dreissigfache Konzentrierung der frischen Knoblauchzehen erzielt worden.

Durch Vermischen mit trockenem Knoblauchpulver kann ein stabiles standardisiertes Arzneimittel in Tabletten-, Dragee- oder Kapselform hergestellt werden.

Das trockene Drogenpulver kann auch durch Zugabe von Alliinase ersetzt werden.

### Beispiel 4

1 kg Herba spartii scop. (Besenginster), grob gemahlen, wurde mit 500 ml Ethanol 96%ig durchfeuchtet und über Nacht stehen gelassen.

Am nächsten Morgen wurde die feuchte Droge in ein Perkolationsrohr gefüllt und dann mit 7,5 Liter 96 %igem Ethanol bei Raumtemperatur perkoliert. Das Perkolat wurde durch Zusatz von 0,3 kg Gelatinepulver entwässert und durch Filtration von der wasserhaltigen Gelatine befreit. Das Filtrat wurde bei max. 40°C und vermindertem Druck vom Lösungsmittel befreit. Erhalten wurden 30 g mit 24 Gew.-% Spartin. Die mit Ethanol extrahierte Droge wurde mit 8 Liter heissem Wasser von 90°C im Rührverfahren extrahiert und wie unter Beispiel 1 beschrieben weiter behandelt. Erhalten wurden 110 g Trockenextrakt.

## Patentansprüche

1. Verfahren zur Herstellung eines die wasserdampfflüchtigen und andere lipophile Inhaltsstoffe enthaltenden Teilextraktes aus Heil- und/oder Gewürzpflanzen oder Teilen davon, welche im frischen oder getrockneten Zustand eingesetzt werden können, dadurch gekennzeichnet, dass man
a) die genannten Pflanzen oder Teile davon mit wenigstens einem organischen Lösungsmittel vermischt und auf übliche Weise extrahiert, dann entweder
b) 1) zu dieser Extraktionsmischung wenigstens ein Trocknungsmittel hinzugibt, um der organischen Phase das Wasser zu entziehen, und anschliessend die getrocknete organische Phase von den pflanzlichen Feststoffen und dem Trocknungsmittel abtrennt, oder
b) 2) die pflanzlichen Feststoffe von der organischen Phase abtrennt, dann dieser organischen Phase wenigstens ein Trocknungsmittel hinzugibt, um das Wasser zu entziehen, und anschliessend die getrocknete organische Phase vom Trocknungsmittel abtrennt, und
c) das oder die organische(n) Lösungsmittel abdestilliert, und so ein lipophiles Konzentrat erhält.

2. Verfahren nach Anspruch 1, dadurch gekennzeichnet, dass man im Falle der Verwendung von frischen Pflanzen oder Teilen davon diese unter dem genannten organischen Lösungsmittel zerkleinert.

3. Verfahren nach einem der Ansprüche 1 bis 2, dadurch gekennzeichnet, dass man dem organischen Lösungsmittel schon vor Beginn der Extraktion oder während der Extraktion ein Trocknungsmittel hinzugibt.

4. Verfahren nach einem der Ansprüche 1 bis 3, dadurch gekennzeichnet, dass die lipophilen Inhaltsstoffe wenigstens einen Bestandteil, ausgewählt aus
- pharmazeutisch aktiven Substanzen
- Harzen
- geruchs- und geschmacksgebenden Verbindungen und
- wasserdampfflüchtigen Substanzen, wie z.B.
aetherische Oele
Naphthochinon-Derivate und
Alkaloide,
umfassen.

5. Verfahren nach einem der Ansprüche 1 bis 4, dadurch gekennzeichnet, dass das organische Lösungsmittel ein polares Lösungsmittel ist und ausgewählt ist aus
- einem Alkohol, insbesondere einem C₁ bis C₄-Alkohol, vorzugsweise Aethanol oder Methanol,
- einem Keton, insbesondere einem C₃ bis C₅-Keton, vorzugsweise Aceton, und
- einem Ester, insbesondere einem Alkylacetat, wobei der Alkylrest vorzugsweise 1 bis 4 C-Atome hat.

6. Verfahren nach einem der Ansprüche 1 bis 5, dadurch gekennzeichnet, dass die getrocknete organische Phase im wesentlichen wasserfrei ist, und insbesondere nicht mehr als 2 Gew.-% Wasser enthält.

7. Verfahren nach einem der Ansprüche 1 bis 6, dadurch gekennzeichnet, dass das Trocknungsmittel ausgewählt ist aus pflanzlichen oder tierischen oder chemischen, wasseraufnehmenden Stoffen, wie z.B. Traganth, Gelatine, wasserfreies Natriumsulfat, wasserfreies Magnesiumsulfat, wasserfreies Calciumchlorid, Molekularsiebe.

8. Verfahren nach einem der Ansprüche 1 bis 7, dadurch gekennzeichnet, dass das Abdestillieren des oder der Lösungsmittel(s) unter Vakuum bei Temperaturen bis zu maximal 60°C erfolgt.

9. Verfahren nach einem der Ansprüche 1 bis 8, dadurch gekennzeichnet, dass die zu extrahierenden Pflanzen ausgewählt sind aus Acorus calamus L. (Rhizom)
Allium - Arten (z.B.:A, cepa L., A. ursinum L., A. sativum L.: Bulbus)
Alpinia officinarum Hance. (Rhizom)
Anethum graveolens L. (Fructus)
Angelica archangelica L. div. Subspec. (Rhizoma)
Anthemis nobilis L. (Chamomilla romana, Herba)
Apium graveolens L. (Fructus)
Arctium major Gaertn. (Radix)
Arnica montana L. (Flos)
Artemisia absinthium L. (Herba)
Artemisia dracunculus L. (Herba)
Brassica nigra [L.] Koch (Semen)
Carum carvi L. (Fructus)
Chrysanthemum vulgare Asch. (Herba)
Cinnamomum camphora, div. Species (Cortex)
Cinnamomum ceylanicum Nees. (Cortex)
Citrus: div. Species (Folium, Flavedo, Fruct.)
Copaifera reticulata Duke (Balsam)
Coriandrum sativum L. (Fructus)
Cuminum cyminum L. (Fructus)
Curcuma zedoaria [Bergius] Roxb. und Unterarten (Rhizoma)
Cusparia officinalis [Willd.] Eng. (Cortex)
Dipterocarpus turbinatus Gaertn. (Bulsamum)
Drosera - Arten (D. rotundifolia L., D.ramentacea Burch; Herba)
Elettaria cardamomum [L.] White et Mathon (Fructus)
Eucalyptus globulus Labill. (Folium)
Foeniculum vulgare Miller (Fructus)
Gaultheria procumbens L. (Folium)
Hedoma pulegioides [L.] Pers. (Herbs)
Hibiscus abelmoschus L. (Semen)
Humulus lupulus L. (Flos, Glandulae)
Hysopus officinalis L. (Herba)
Illicium varum Hook.f. (Fructus)
Inula helenium L. (Rhizoma)
Iris pallida Lam. (Rhizoma)
Jasminum grandiflorum L. (Flos)
Laurus nobilis L. (Folium, Fructus)
Lavendula officinalis, div. Subspecies (Flos)
Lawsonia inermis L. (Folium)
Levisticum officinale Koch (Radix)
Matricaria chamomilla L. (Flos)
Melaleuca: div. Arten. (Folium)
Melilotus officinalis [L.] Lam. em. Thuill. (Herba)
Melissa officinalis L. (Herba)
Mentha,alle Arten (Folium)
Myristica fragrans Houttuyn (Arillus, Semen)
Myrtus communis L. (Folium)
Ocimum basilicum L. (Herba)
Ocotea sassafras (Cortex)
Oenanthe aquatica [L.] Poir (Fructus)
Olibanum (Resinum)
Origanum majorana L. und Unterarten (Herba)
Petroselinum criapum [Mill.] Nym. (Fructus, Herba)
Pimenta dioica [L.] Merill (Fructus)
Pimpinella anisum L. (Semen)
Piper angustifolium Ruiz. et Pavon. (Folium)
Pogostemon patchouli Pell. (Folium)
Prunus lauroccrasus L. (Folium)
Rosmarinus officinalis L. und Unterarten (Folium)
Ruta graveolens L. (Herba)
Salvia officinalis L. und Unterarten (Folium)
Santalum album L. (Lignum)
Sarothamnus scoparius [L.] Wimmer (Herba)
Sassafras albidum [Nutt`] Nees (Lignum)
Satureja hortensis L. (Herba)
Syzygium aromaticum Merr. et Perry (Flores, Folium)
Thymus serpyllum L. (Herba)
Thymus vulgaris L. (Herba)
Tilis cordata Mill. und T. platyphyllos Scop. (Flos)
Valeriana officinalis und Unterarten (Radix)
Zingiberis officinale Roscoe (Rhizoma).

10. Verfahren zur Herstellung eines die wasserdampfflüchtigen und andere lipophile sowie die hydrophilen Inhaltsstoffe enthaltenden Vollextraktes aus Heil- und/oder Gewürzpflanzen oder Teilen davon, welche im frischen oder getrockneten Zustand eingesetzt werden können, dadurch gekennzeichnet, dass man
A) die Schritte a) bis c) gemäss dem Verfahren nach einem der Ansprüche 1 bis 9 durchführt, um ein lipophiles Konzentrat zu erhalten, dann
B) die Rückstände gemäss den Schritten b) 1) oder b) 2) mit Wasser oder mit Wasser mischbaren verdünnten organischen Lösungsmitteln extrahiert, und
C) die Feststoffe von der flüssigen Phasen abtrennt,
D) das Wasser und gegebenenfalls das mit Wasser verdünnte organische Lösungsmittel aus dem so erhaltenen Filtrat abdestilliert, und so ein hydrophiles Konzentrat erhält, und
E) das lipophile Konzentrat von Schritt A) mit dem hydrophilen Konzentrat von Schritt D) zu einem Vollextrakt vermischt.

11. Verwendung der gemäss dem Verfahren nach einem der Ansprüche 1 bis 10 erhaltenen Teil- oder Vollextrakte als Heilmittel oder als Gewürz.

## Claims

1. A process for preparing a partial extract containing the volatile in steam components and further lipophilic components of medical plants and/or spice plants or parts thereof, which may be charged in a fresh or dried state, characterized by
a) mixing said plants or parts thereof with at least one organic solvent and extracting in usual manner, then either
b) 1) adding to this extraction mixture at least one drying agent, in order to take away the water from the organic phase, and then separating the dried organic phase from the vegetable solid matter and from the drying agent, or
b) 2) separating the vegetable solid matter from the organic phase, then adding to this organic phase at least one drying agent, in order to take away the water, and then separating the dried organic phase from the drying agent, and
c) distilling the organic solvent(s), and recovering in this way a lipophilic concentrate.

2. The process according to claim 1, characterized in that in the case of using fresh plants or parts thereof these are reduced to small pieces in said organic solvent.

3. The process according to one of claims 1 to 2, characterized by adding a drying agent to the organic solvent already before the beginning of the extraction or during the extraction.

4. The process according to one of claims 1 to 3, characterized in that the lipophilic components comprise at least one component selected from
- pharmaceutical active substances
- resins
- smell and flavor providing compounds and
- substances which are volatile in steam, such as e.g.
ethereal oils
naphthoquinone derivatives and
alkaloids.

5. The process according to one of claims 1 to 4, characterized in that the organic solvent is a polar solvent and is selected from
- an alcohol, especially a C₁ to C₄-alcohol, preferably ethanol or methanol,
- a ketone, especially a C₃ to C₅-ketone, preferably acetone, and
- an ester, especially an alkylacetate, whereby the alkyl residue has preferably from 1 to 4 C-atoms.

6. The process according to one of claims 1 to 5, characterized in that the dried organic phase is essentially free of water, and contains especially not more than 2 % by weight of water.

7. The process according to one of claims 1 to 6, characterized in that the drying agent is selected from vegetable or animal or chemical water-absorbing materials, such as e.g. tragacanth, gelatine, water-free sodium sulfate, water-free magnesium sulfate, water-free calcium chloride, molecular sieves.

8. The process acording to one of claims 1 to 7, characterized in that the distilling of the solvent(s) is realized in vacuum at temperatures up to a maximum of 60°C.

9. The process according to one of claims 1 to 8, characterized in that the plants to be extracted are selected from:
Acorus calamus (Rhizom)
Allium-species, (e.g. A. cepa L., A. ursinum L., A. sativum L.:Bulbus)
Alpinia officinarum Hance (Rhizom)
Anethum graveolens L. (Fructus)
Angelica archangelica L., various subspec. (Rhizoma)
Anthemis nobilis L. (Chamomilla romana, Herba)
Apium graveolens L. (Fructus)
Arctium major Gaertn. (Radix)
Arnica montana L. (Flos)
Artemisia absinthium L. (Herba)
Artemisia dracunculus L. (Herba)
Brassica nigra (L.) Koch (Semen)
Carum carvi L. (Fructus)
Chrysanthemum vulgare Asch. (Herba)
Cinnamomum camphora, various species (Cortex)
Cinnamomum ceylanicum Nees. (Cortex)
Citrus: various species (Folium, Flavedo, Fruct.)
Copaifera reticulata Duke (Balsam)
Coriandrum sativum L. (Fructus)
Cuminum cyminum L. (Fructus)
Curcuma zedoaria [Bergius] Roxb. and subspecies (Rhizoma)
Cusparia officinalis (Willd.) Eng. (Cortex)
Dipterocarpus turbinatus Gaertn. (Balsamum)
Drosera species (D. rotundifolia L., D.ramentacea Burch; Herba)
Elettaria cardamomum (L.) White et Mathon (Fructus)
Eucalyptus globulus Labill. (Folium)
Foeniculum vulgare Miller (Fructus)
Gaultheria procumbens L. (Folium)
Hedoma pulegioides (L.) Pers. (Herba)
Hibiscus abelmoschus L. (Semen)
Humulus lupulus L. (Flos, Glandulae)
Hysopus officinalis L. (Herba)
Illicium verum Hook. f. (Fructus)
Inula helenium L. (Rhizoma)
Iris pallida Lam. (Rhizoma)
Jasminum grandiflorum L. (Flos)
Laurus nobilis L. (Folium, Fructus)
Lavendula officinalis, various subspecies (Flos)
Lawsonia inermis L. (Folium)
Levisticum officinale Koch (Radix)
Matricaria chamomilla L. (Flos)
Melaleuca; various species (Folium)
Melilotus officinalis (L.) Lam. em. Thuill. (Herba)
Melissa officinalis L. (Herba)
Mentha, all species (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olibanum (Resinum)
Origanum majorana L. and subspecies (Herba)
Petroselinum crispum (Mill.) Nym. (Fructus, Herba)
Pimenta dioica (L.) Merill (Fructus)
Pimpinella anisum L. (Semen)
Piper angustifolium Ruiz. et Pavon. (Folium)
Pogostemon patchouli Pell. (Folium)
Prunus laurocerasus L. (Folium)
Rosmarinus officinalis L. and subspecies (Folium)
Ruta graveolens L. (Herba)
Salvia officinalis L. and subspecies (Folium)
Santalum album L. (Lignum)
Sarothamnus scoparius (L.) Wimmer (Herba)
Sassafras albidum (Nutt.) Nees (Lignum)
Satureja hortensis L. (Herba)
Syzygium aromaticum Merr. et Perry (Flores, Folium)
Thymus serpyllum L. (Herba)
Thymus vulgaris L. (Herba)
Tilia cordata Mill. and T. platyphyllos Scop. (Flos)
Valeriana officinalis and subspecies (Radix)
Zingiberis officinale Roscoe (Rhizoma).

10. A process for preparing a complete extract containing the volatile in steam and further lipophilic components as well as the hydrophilic components of medical plants and/or spice plants or parts thereof, which may be charged in a fresh or dried state, characterized by:
A) carrying out the steps a) to c) according to the process according to one of claims 1 to 9, in order to obtain a lipophilic concentrate, then
B) extracting the residues according to steps b) 1) or b) 2) with water or with water miscible diluted organic solvents, and
C) separating the solid matter from the liquid phase,
D) distilling the water and occasionally the with water diluted organic solvent from the so obtained filtrate, and obtaining in this way a hydrophilic concentrate, and
E) mixing the lipophilic concentrate from step A) with the hydrophilic concentrate from step D) to a complete extract.

11. Use of the partial extracts or complete extracts obtained according to the process according to one of claims 1 to 10 as a remedy or as a spice.

## Revendications

1. Procédé de préparation d'un extrait partiel contenant des constituants volatils sous l'effet de la vapeur d'eau et des autres constituants lipophiles au départ de plantes médicinales et/ou condimentaires ou de parties de celles-ci qui peuvent être utilisées à l'état frais ou séché, caractérisé en ce que
a) on mélange les plantes précitées ou les parties de celles-ci avec au moins un solvant organique et on réalise l'extraction de la manière usuelle, puis
b) 1) soit on ajoute à ce mélange d'extraction au moins un agent de séchage afin d'éliminer l'eau de la phase organique et on sépare ensuite la phase organique séchée des matières solides végétales et de l'agent de séchage, et
b) 2) soit on sépare les matières solides végétales de la phase organique puis on ajoute à cette phase organique au moins un agent de séchage afin d'éliminer l'eau et on sépare ensuite la phase organique séchée de l'agent de séchage, et
c) on élimine par distillation le ou les solvant(s) organique(s) et l'on obtient ainsi un concentré lipophile.

2. Procédé selon la revendication 1, caractérisé en ce que, en cas d'utilisation de plantes fraîches ou de parties de celles-ci, on broie ces matières premières dans ledit solvant organique.

3. Procédé selon l'une des revendications 1 à 2, caractérisé en ce que l'on ajoute un agent de séchage au solvant organique déjà avant le début de l'extraction ou pendant celle-ci.

4. Procédé selon l'une des revendications 1 à 3, caractérisé en ce que les constituants lipophiles comprennent au moins un composant choisi parmi les substances suivantes:
- substances à effet pharmaceutique
- résines
- composés odorants et aromatiques et
- substances volatiles avec la vapeur d'eau comme, par exemple
les huiles éthérées
dérivés de la naphtoquinone et
alcaloides.

5. Procédé selon l'une des revendications 1 à 4, caractérisé en ce que le solvant organique est un solvant polaire et que celui-ci est choisi parmi les substances suivantes:
- un alcool et, en particulier, un alcool en C₁ à C₄ et de préférence de l'éthanol ou du méthanol,
- une cétone et, en particulier, une cétone en C₃ à C₅ et de préférence de l'acétone, et
- un ester et, en particulier, un acétate d'alkyle, tandis que le radical alkyle comprend de préférence 1 à 4 atomes de carbone.

6. Procédé selon l'une des revendications 1 à 5, caractérisé en ce que la phase organique séchée est essentiellement anhydre et ne contient en particulier pas plus de 2 % en poids d'eau.

7. Procédé selon l'une des revendications 1 à 6, caractérisé en ce que l'agent de séchage est choisi parmi les substances végétales, animales ou chimiques absorbant de l'eau comme, par exemple, la gomme adragante, la gélatine, le sulfate de sodium anhydre, le sulfate de magnésium anhydre, le chlorure de calcium anhydre, les tamis moléculaires.

8. Procédé selon l'une des revendications 1 à 7, caractérisé en ce que l'élimination par distillation du ou des solvant(s) est effectuée sous vide à des températures atteignant au maximum 60°C.

9. Procédé selon l'une des revendications 1 à 8, caractérisé en ce que les plantes à extraire sont choisies parmi les suivantes:
Acorus calamus L. (Rhizomes)
Allium (genre) (p. ex. A. cepa L., A. ursinum L., A. sativum L.:Bulbus)
Alpinia officinarum Hance (Rhizomes)
Anethum graveolens L. (Fructus)
Angelica archangelica L. div. sous-espèces (Rhizomes)
Anthemis nobilis L. (Chamomilla romana, Herba)
Apium graveolens L. (Fructus)
Arctium major Gaertn. (Radix)
Arnica montana L. (Flos)
Artemisia absinthium L. (Herba)
Artemisia dracunculus L. (Herba)
Brassica nigra (L.) Koch (Semen)
Carum carvi L. (Fructus)
Chrysanthemum vulgare Asch. (Herba)
Cinnamomum camphora, div. espèces (Cortex)
Cinnamomum ceylanicum Nees. (Cortex)
Citrus: div. espèces (Folium, Flavedo, Fruct.)
Copaifera reticulata Duke (Balsam)
Coriandrum sativum L. (Fructus)
Cuminum cyminum L. (Fructus)
Curcuma zedoaria [Bergius] Roxb. et sous-espèces (Rhizomes)
Cusparia officinalis (Willd.) Eng. (Cortex)
Dipterocarpus turbinatus Gaertn. (Balsamum)
Drosera species (D. rotundifolia L., D.ramentacea Burch; Herba)
Elettaria cardamomum (L.) White et Mathon (Fructus)
Eucalyptus globulus Labill. (Folium)
Foeniculum vulgare Miller (Fructus)
Gaultheria procumbens L. (Folium)
Hedoma pulegioides (L.) Pers. (Herba)
Hibiscus abelmoschus L. (Semen)
Humulus lupulus L. (Flos, Glandulae)
Hysopus officinalis L. (Herba)
Illicium verum Hook. f. (Fructus)
Inula helenium L. (Rhizoma)
Iris pallida Lam. (Rhizoma)
Jasminum grandiflorum L. (Flos)
Laurus nobilis L. (Folium, Fructus)
Lavendula officinalis, div. sous-espèces (Flos)
Lawsonia inermis L. (Folium)
Levisticum officinale Koch (Radix)
Matricaria chamomilla L. (Flos)
Melaleuca; div. espèces (Folium)
Melilotus officinalis (L.) Lam. em. Thuill. (Herba)
Melissa officinalis L. (Herba)
Mentha, toutes espèces (Folium)
Myristica fragrans Houttuyn (Arillus, Semen)
Myrtus communis L. (Folium)
Ocimum basilicum L. (Herba)
Ocotea sassafras (Cortex)
Oenanthe aquatica (L.) Poir (Fructus)
Olibanum (Resinum)
Origanum majorana L. et sous-espèces (Herba)
Petroselinum crispum (Mill.) Nym. (Fructus, Herba)
Pimenta dioica (L.) Merill (Fructus)
Pimpinella anisum L. (Semen)
Piper angustifolium Ruiz. et Pavon. (Folium)
Pogostemon patchouli Pell. (Folium)
Prunus laurocerasus L. (Folium)
Rosmarinus officinalis L. et sous-espèces (Folium)
Ruta graveolens L. (Herba)
Salvia officinalis L. et sous-espèces (Folium)
Santalum album L. (Lignum)
Sarothamnus scoparius (L.) Wimmer (Herba)
Sassafras albidum (Nutt.) Nees (Lignum)
Satureja hortensis L. (Herba)
Syzygium aromaticum Merr. et Perry (Flores, Folium)
Thymus serpyllum L. (Herba)
Thymus vulgaris L. (Herba)
Tilia cordata Mill. et T.platyphyllos Scop. (Flos)
Valeriana officinalis et sous-espèces (Radix)
Zingiberis officinale Roscoe (Rhizoma).

10. Procédé de préparation d'un extrait complet contenant les composants volatils avec la vapeur d'eau et les autres composants lipophiles et hydrophiles au départ de plantes médicinales et/ou condimentaires ou de parties de celles-ci qui peuvent être utilisées à l'état frais ou séché, caractérisé en ce que
A) l'on réalise les étapes a) à c) conformément aux procédés décrits dans l'une des revendications 1 à 9, afin d'obtenir un concentré lipophile, puis
B) l'on extrait les résidus suivant les étapes b) 1) ou b) 2) avec de l'eau ou des solvants organiques dilués, solubles dans l'eau, et
C) l'on sépare les matières solides de la phase liquide,
D) l'on élimine par distillation de ce filtrat si obtenue l'eau et, éventuellement, le solvant organique dilué par de l'eau pour obtenir ainsi un concentré hydrophile, et
E) l'on mélange le concentré lipophile de l'étape A) avec le concentré hydrophile de l'étape D) pour obtenir un extrait complet.

11. Utilisation des extraits partiels ou complets obtenus grâce aux procédés décrits dans l'une des revendications 1 à 10 comme médicament ou comme condiment.
